(19) Europäisches Patentamt
European Patent Office
Office européen des brevets

(11) Veröffentlichungsnummer: **0 601 477 A1**

(12) # EUROPÄISCHE PATENTANMELDUNG

(21) Anmeldenummer: **93119421.1**

(51) Int. Cl.[5]: **C07C 259/06**, A01N 37/28

(22) Anmeldetag: **02.12.93**

(30) Priorität: **08.12.92 DE 4241211**

(43) Veröffentlichungstag der Anmeldung: **15.06.94 Patentblatt 94/24**

(84) Benannte Vertragsstaaten: **AT BE CH DE DK ES FR GB GR IE IT LI NL**

(71) Anmelder: **HOECHST SCHERING AGREVO GmbH**
**Gerichtstr. 27**
**D-13342 Berlin(DE)**

(72) Erfinder: **Vermehren, Jan, Dr.**
**Im Hinterlenzen 33**
**D-65510 Idstein/Taunus(DE)**
Erfinder: **Heubach, Günther, Dr.**
**Luisenstrasse 15**
**D-65779 Kelkheim/Taunus(DE)**
Erfinder: **Braun, Peter, Dr.**
**Pfarrer-Dorn-Strasse 13**
**D-55127 Mainz(DE)**
Erfinder: **Sachse, Burkhard, Dr.**
**An der Ziegelei 30**
**D-65779 Kelkheim/Taunus(DE)**

(54) **Substituierte Zimtsäurehydroxyamide, Verfahren zu ihrer Herstellung, diese enthaltende Mittel und ihre Verwendung.**

(57) Die Erfindung betrifft neue N-Alkoxy-zimtsäureamide der allgemeinen Formel I

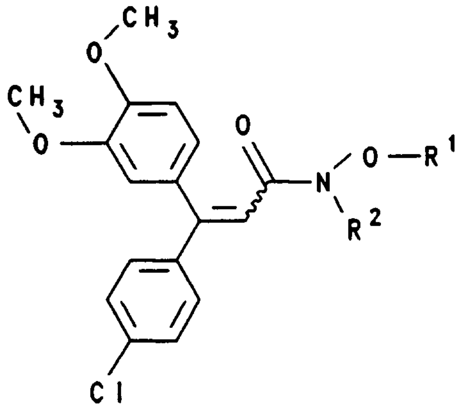

(I)

in der $R^1$ und $R^2$ Alkyl, Alkenyl, Alkinyl, Alkoxyalkyl, Alkoxyalkenyl, Alkoxyalkinyl, Trialkylsilylalkinyl oder Arylalkyl bedeuten, wobei Aryl gegebenenfalls durch Alkyl, Alkoxy, Haloalkyl, Haloalkoxy, Alkoxycarbonyl, Halogen, Cyano, Nitro, Alkylsulfonyl, Phenyl und/oder Phenoxy substituiert ist, Verfahren zu ihrer Herstellung, diese enthaltende fungizide Mittel und ihre Verwendung als Fungizide.

EP 0 601 477 A1

Die Erfindung betrifft neue N-Alkoxy-zimtsäureamide der allgemeinen Formel I

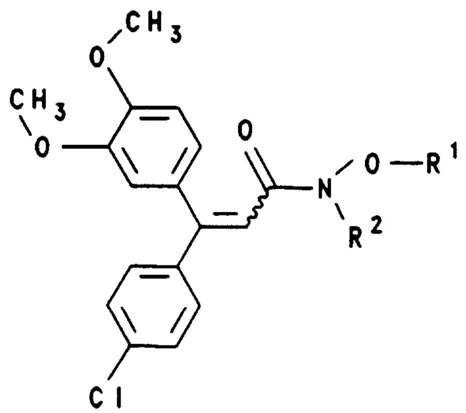

(I)

in der $R^1$ und $R^2$ Alkyl, Alkenyl, Alkinyl oder gegebenenfalls substituiertes Arylalkyl bedeutet, Verfahren zu ihrer Herstellung, diese enthaltende fungizide Mittel und ihre Verwendung als Fungizide.

Aus der EP-A-208999 sind N-Alkoxy-zimtsäureamide,wie z.B. das N-Methoxy-3-(4-methoxy-3-methylphenyl)-N-methyl-zimtsäureamid und deren Verwendung als Fungizide bekannt. Ihre Wirkung ist jedoch oft nicht ausreichend.

Es wurden neue Zimtsäurehydroxyamide mit ausgezeichneter fungizider Wirkung gefunden.

Die Erfindung betrifft daher Verbindungen der Formel I,

in der

$R^1$ und $R^2$ unabhängig voneinander $C_1$-$C_6$-Alkyl, $C_2$-$C_6$-Alkenyl, $C_2$-$C_6$-Alkinyl, $C_1$-$C_4$-Alkoxy-$C_1$-$C_6$-alkyl, $C_1$-$C_4$-Alkoxy-$C_2$-$C_6$-alkenyl, $C_1$-$C_4$-Alkoxy-$C_2$-$C_6$-alkinyl, Tri-($C_1$-$C_4$-alkyl)-silyl-$C_2$-$C_6$-alkinyl oder Aryl-$C_1$-$C_2$-alkyl, wobei Aryl gegebenenfalls bis zu vierfach durch gleiche oder verschiedene Reste aus der Reihe $C_1$-$C_4$-Alkyl, $C_1$-$C_4$-Alkoxy, $C_1$-$C_4$-Haloalkyl, $C_1$-$C_4$-Haloalkoxy, $C_1$-$C_4$-Alkoxycarbonyl, Halogen, Cyano, Nitro, $C_1$-$C_4$-Alkylsulfonyl, Phenyl und Phenoxy substituiert ist,

bedeuten,

Dabei können die Alkyl-, Alkenyl- oder Alkinylreste sowohl geradkettig als auch verzweigt sein; die Mehrfachbindungen können sowohl end- wie auch mittelständig sein. Entsprechendes gilt für davon abgeleitete Reste, wie Alkoxy usw.. Halogen bedeutet F, Cl, Br, I, bevorzugt F, Cl, Br. Die Vorsilbe "Halo" in der Bezeichnung eines Substituenten bedeutet hier und im folgenden, daß Halogen als Substituent einfach oder mehrfach bei gleicher oder verschiedener Bedeutung auftreten kann. Erläuternd, aber nicht einschränkend seien als Beispiele für Haloalkyl genannt: $CF_3$, $CHF_2$, $CH_2Br$, $CHCl_2$, $CF_2CHF_2$, $CCl_3$, $CCl_2F$, $CF_2CF_2CF_3$, $CF_2CHFCF_3$, $CH_2CF_3$ und $(CF_2)_3CF_3$. Entsprechendes gilt für davon abgeleitete Reste, wie Haloalkoxy.

Aryl steht vorzugsweise für $C_6$-$C_{12}$-Aryl, wie Phenyl, Naphthyl oder Biphenylyl, insbesondere Phenyl.

Bevorzugt unter den Verbindungen der Formel I sind solche, worin $R^1$ und $R^2$ unabhängig voneinander $C_1$-$C_4$-Alkyl, $C_2$-$C_4$-Alkenyl, $C_2$-$C_4$-Alkinyl, $C_1$-$C_4$-Alkoxy-$C_1$-$C_4$-alkyl, $C_1$-$C_4$-Alkoxy-$C_2$-$C_4$-alkenyl, $C_1$-$C_4$-Alkoxy-$C_2$-$C_4$-alkinyl, Tri-($C_1$-$C_2$-Alkyl)-silyl-$C_2$-$C_4$-alkinyl oder Aryl-methyl, wobei Aryl gegebenenfalls bis zu vierfach substituiert ist durch gleiche oder verschiedene Reste aus der Reihe $C_1$-$C_4$-Alkyl, $C_1$-$C_2$-Alkoxy, $C_1$-$C_4$-Haloalkyl, $C_1$-$C_2$-Haloalkoxy, $C_1$-$C_2$-Alkoxycarbonyl, Halogen, Cyano, Nitro, Methylsulfonyl, Phenyl und Phenoxy, bedeuten.

Besonders bevorzugt unter den Verbindungen der Formel I sind solche, worin $R^1$ und $R^2$ unabhängig voneinander $C_1$-$C_2$-Alkyl, $C_2$-$C_3$-Alkenyl, $C_2$-$C_3$-Alkinyl, $C_1$-$C_2$-Alkoxy-$C_1$-$C_2$-alkyl, $C_1$-$C_2$-Alkoxy-$C_2$-$C_3$-alkenyl, $C_1$-$C_2$-Alkoxy-$C_2$-$C_3$-alkinyl, Trimethylsilyl-$C_2$-$C_3$-alkinyl oder Benzyl bedeuten.

Die neuen Verbindungen der Formel I können bei ihrer Herstellung aufgrund der C = C-Doppelbindung als E/Z-Isomerengemisch anfallen, die in üblicher Weise, z.B durch Kristallisation oder Chromatographie in die einzelnen Komponenten getrennt werden können. Weiterhin können die Verbindungen der Formel I zum Teil ein oder mehrere asymmetrische Kohlenstoff- oder Heteroatome aufweisen. Es können dann Racemate und Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer optisch aktiven Säure, Trennung der diastereomeren Salze und Freisetzung der reinen

Enantiomeren mittels einer Base. Sowohl die einzelnen isomeren Verbindungen als auch ihre Gemische werden von der Erfindung erfaßt und sind als fungizide Wirkstoffe brauchbar.

Man erhält die Verbindungen nach an sich bekannten Verfahren durch:

a) Umsetzung der 3-(4-Chlorphenyl)-3-(3,4-dimethoxyphenyl)-acrylsäure der Formel II oder durch Umsetzung

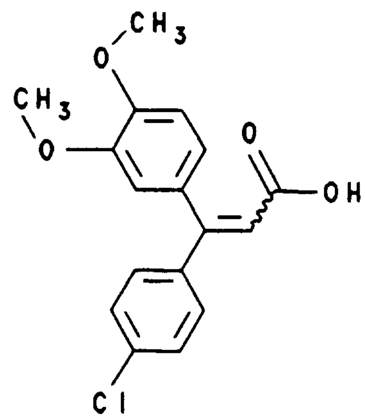

(II)

eines gegebenenfalls in situ hergestellten reaktionsfähigen Derivates davon mit einem Hydroxyaminderivat der Formel III,

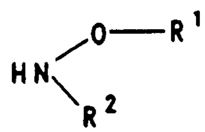

(III)

in dem $R^1$ und $R^2$ wie zuvor definiert sind. Zusätzlich können $R^1$ und/oder $R^2$ Wasserstoff bedeuten.

Das Verfahren stellt somit die Acylierung einer Verbindung der Formel III mit einer Carbonsäure der Formel II dar, wobei die Umsetzung vorteilhaft in Gegenwart eines die Säure II aktivierenden oder eines wasserentziehenden Mittels oder aber mit reaktiven Derivaten der Carbonsäure der Formel II oder der Verbindung der Formel III gearbeitet wird.

Als gegebenenfalls im Reaktionsgemisch hergestellte Derivate einer Carbonsäure der Formel II kommen beispielsweise ihre Alkyl-, Aryl-, Arylalkylester wie der Methyl-, Ethyl-, Phenyl- oder Benzylester, ihre Imidazolide, ihre Säurehalogenide wie das Säurechlorid oder -bromid, ihre Anhydride, ihre gemischten Anhydride mit aliphatischen oder aromatischen Carbon-, Sulfonsäuren oder mit Kohlensäureestern, z.B. mit der Essigsäure, der Propionsäure, der p-Toluolsulfonsäure oder der O-Ethyl-, oder O-Isobutylkohlensäure, oder ihre N-Hydroximidester in Betracht.

Als säureaktivierende und/oder wasserentziehende Mittel kommen beispielsweise ein Chlorkohlensäureester wie Chlorameisensäureethylester, Chlorameisensäureisobutylester, Phosphorpentoxid, N,N'-Dicyclohexylcarbodiimid, N,N'-Carbonyldiimid, N,N'-Carbonyldiimidazol oder N,N'-Thionyldiimid in Betracht.

Die Umsetzung wird zweckmäßigerweise in einem inerten Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril, N-Methylpyrrolidin oder Dimethylformamid, gegebenenfalls in Gegenwart einer anorganischen Base wie Natriumcarbonat oder einer tertiären organischen Base wie Triethylamin, N-Methylmorpholin oder Pyridin, welche gleichzeitig als Lösungsmittel dienen kann, und gegebenenfalls in Gegenwart eines säureaktivierenden Mittels bei Temperaturen zwischen -78 °C und 120 °C, vorzugsweise jedoch bei Temperaturen zwischen -78 °C und der Siedetemperatur des Reaktionsgemisches, durchgeführt. Hierbei braucht ein gegebenfalls im Reaktionsgemisch entstandenes reaktionsfähiges Derivat einer Verbindung der allgemeinen Formel II oder III nicht isoliert zu werden; ferner kann die Umsetzung auch in einem Überschuß der eingesetzten Verbindung der allgemeinen Formel III als Lösungsmittel durchgeführt werden.

Erfindungsgemäß erhaltene E/Z-Isomerengemische können gewünschtenfalls anschließend nach üblichen Methoden in die entsprechenden E- und Z-Isomeren getrennt werden.

b) Verbindungen der Formel I sind auch darstellbar durch Umsetzung eines Ketons der Formel IV mit einem Phosphonessigsäureamid der Formel V,

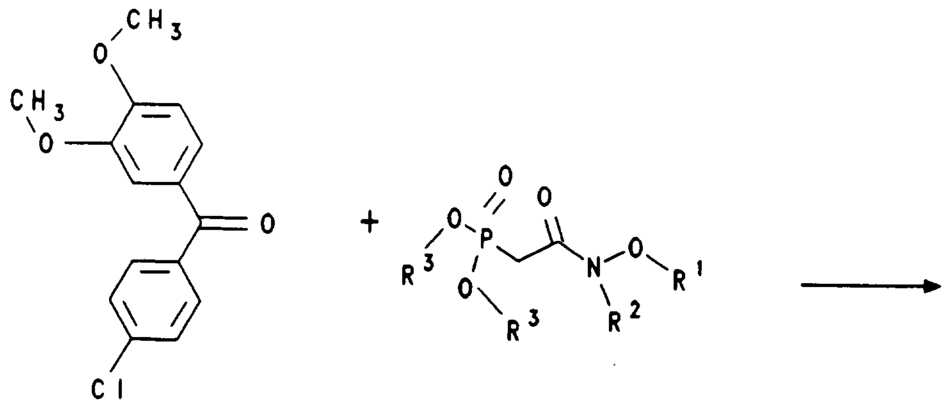

(IV) (V) (I)

in dem $R^1$ und $R^2$ wie oben definiert sind und $R^3$ für einen aliphatischen Rest, vorzugsweise für einen niederen Alkylrest insbesondere mit bis zu 6 C-Atomen steht, nach dem Verfahren von Wittig und Horner.

Verbindung IV ist aus der DE-A-3643403 bekannt.

c) Verbindungen der Formel I sind auch darstellbar durch Umsetzung eines N-Alkoxyzimtsäureamids der Formel VI mit einem Alkylierungsmittel der Formel VII, wobei die Reste $R^1$ und $R^2$ wie oben definiert sind und X Halogen oder Alkoxysulfonyl, vorzugsweise mit bis zu 6 C-Atomen bedeutet, in Gegenwart einer Base wie Alkalimetallhydriden, insbesondere Natriumhydrid oder einem Alkalimetallalkoholat wie z.B Kalium-tert.butylat.

Die Umsetzung wird zweckmäßigerweise in einem Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril, N-Methylpyrrolidin oder Dimethylformamid, bei Temperaturen zwischen -40 °C und 180 °C, bevorzugt zwischen -40 °C und dem Siedepunkt des Reaktionsgemisches durchgeführt.

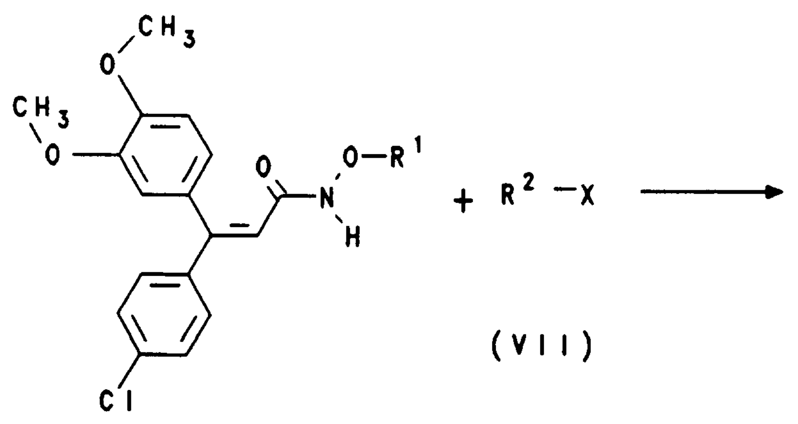

I

(VII)

(VI),

Die Verbindungen der Formel VI lassen sich wie auf dem Weg a) beschrieben herstellen.

d) Verbindungen der Formel I sind auch herstellbar durch Umsetzung eines Ketons der Formel IV mit einem Acethydroxamsäurederivat der allgemeinen Formel IX, wobei die Reste $R^1$ und $R^2$ wie oben definiert sind, in Gegenwart einer starken Base.

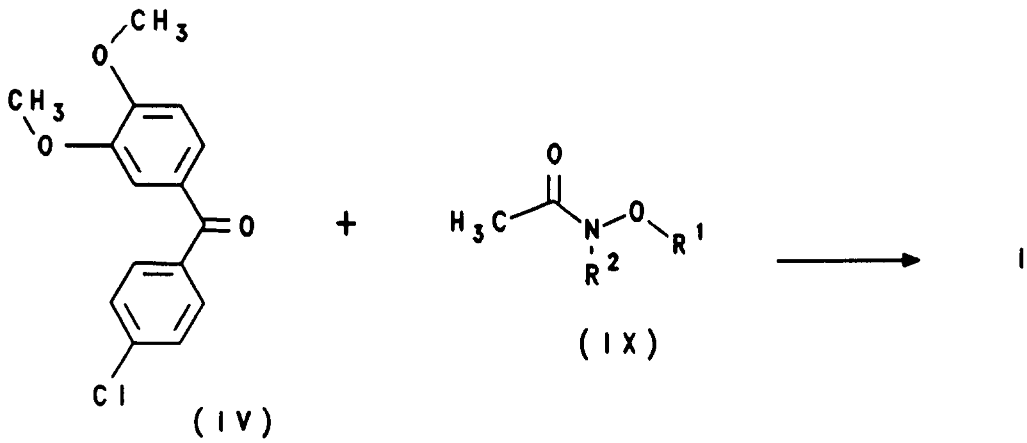

(IV)

(IX)

Geeignete Basen sind z.B. Metallhydroxide, Metallcarbonate, Metallalkoholate und Metallsalze von Monoalkylcarbonaten oder Gemische dieser Basen. Die Reaktion wird günstigerweise in einem inerten Verdünnungsmittel wie Toluol, Xylol, Diethylether, Tetrahydrofuran, Diglyme, N,N-Dimethylformamid oder N-Methylpyrrolidon durchgeführt. In Abhängigkeit von der Reaktivität der Reagenzien kann die Reaktion bei Temperaturen zwischen 25 °C und dem Siedepunkt der Reaktionsmischung durchgeführt werden. Ein 1 bis 3-facher Überschuß an Base kann von Vorteil sein, ebenso begünstigt ein 1-3-facher Überschuß der Verbindung der Formel IX die Reaktion.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt verschiedene wirtschaftlich bedeutende, phytopathogene Pilze, wie z.B. Phytophthora infestans und Plasmopara viticola.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Gegenstand der Erfindung sind auch Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage: Spritzpulver (WP), emulgierbare Konzentrate (EC), wäßrige Dispersionen auf Öl- oder Wasserbasis (SC), Suspoemulsionen (SC), Stäubemittel (DP), Beizmittel, Granulate in Form von wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:

Winnacker-Küchler, "Chemische Technologie", Band 7, C. Hauser Verlag München, 4. Aufl. 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:

Watkins, "Handbook of Insecticide Dust Diluents and Carrier", 2nd Ed., Darland Books, Caldwell N.J.; H.v.Olphen, "Introduction to Clay Colloid Chemistry, 2nd Ed., J. Wiley & Sons, N.Y.; Marsden, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte

Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:
Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit oder Pyrophyllit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingen wie Temperatur, Feuchtigkeit u. a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z.B folgende Produkte zu nennen:

Aldimorph, Andoprim (PM213), Anilazine, BAS 480F, BAS 490F, Benalaxyl, Benodanil, Benomyl, Binapacryl, Bitertanol, Bromuconazol, Buthiobate, Captafol, Captan, Carbendazim, Carboxin, CGA 173506, Chlobenzthiazone, Chlorthalonil, Cymoxanil, Cyproconazole, Cyprofuram, Dichlofluanid, Dichlomezin, Diclobutrazol, Diethofencarb, Difenconazol (CGA 169374), Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Fluazinam, Fluobenzimine, Fluquinconazole, Fluorimide, Flusilazole, Flutolanil, Flutriafol, Folpet, Fosetylaluminium, Fuberidazole, Fulsulfamide, Furalaxyl, Furconazol (LS 840606), Furmecyclox, Guazatine, Hexaconazole, ICI A 5504, Imazalil, Imibenconazole, Iprobenfos, Iprodione, Isoprothiolane, Kupferverbindungen wie Cu-oxychlorid, Oxine-Cu, Cu-oxide, Mancozeb, Maneb, Mepanipyrim, Metconazol, Mepronil, Metalaxyl, Methasulfocarb, Methfuroxam, Myclobutanil, Nabam, Nitrothalidopropyl, Nuarimol, Ofurace, Oxadixyl, Oxycarboxin, Penconazol, Pencycuron, PP 969, Probenazole, Propineb, Prochloraz, Procymidon, Propamocarb, Propiconazol, Prothiocarb, Pyracarbolid, Pyrazophos, Pyrifenox, Pyroquilon, Rabenzazole, RH7592, Schwefel, Tebuconazole, TF 167, Thiabendazole, Thicyofen, Thiofanatemethyl, Thiram, Tolclofos-methyl, Tolylfluanid, Triadimefon, Triadimenol, Tricyclazole, Tridemorph, Triflumizol, Triforine, Validamycin, Vinchlozolin, Zineb, Natriumdodecylsulfonate, Natrium-dodecylsulfat, Natrium-C13/C15-alkoholethersulfonat, Natrium-cetostearylphosphatester, Dioctyl-natriumsulfosuccinat, Natrium-isopropylnaphthalenesulfonat, Natriummethylenebisnaphthalenesulfonat, Cetyltrimethyl-ammoniumchlorid, Salze von langkettigen primären, sekundären oder tertiären Aminen, Alkylpropyleneamine, Lauryl-pyrimidiniumbromid, ethoxilierte quarternierte Fettamine, Alkyl-dimethyl-benzyl-ammoniumchlorid und 1-Hydroxyethyl-2-alkyl-imidazolin.

Die oben genannten Kombinationspartner stellen bekannte Wirkstoffe dar, die zum großen Teil in CH.R Worthing, U.S.B. Walker, The Pesticide Manual, 7. Auflage (1983), British Crop Protection Council beschrieben sind.

Darüber hinaus kann der erfindungsgemäße Wirkstoff in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u. a. Bevorzugte Mischungspartner sind:

1. Aus der Gruppe der Phosphorverbindungen

Acephate, Azamethiphos, Azinphos-ethyl, Azinphosmethyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, O,O-1,2,2,2-Tetrachlorethylphosphorthioate (SD 208 304), Dimethoate, Disulfoton, EPN, Ethion, Ethoprophos, Etrimfos, Famphur, Fenamiphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion.

2. Aus der Gruppe der Carbamate

Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)-carbamate, Oxamyl, Pirimicarb, Propoxur, Thiodicarb, Thiofanox, Ethyl 4,6,9-triaza-4-benzyl-6,10-dimethyl-8-oxa7-oxo-5,11-dithia-9-dodecenoate (OK 135), 1-Methylthio(ethylideneamino)-N-methyl-N-(morpholinothio)carbamate (UC 51717).

3. Aus der Gruppe der Carbonsäureester

Allethrin, Alphamethrin, 5-Benzyl-3-furylmethyl-(E)-(1R)-cis-2,2-di-methyl-3-(2-oxothiolan-3-ylidenemethyl)cyclopropanecarboxylate, Bioallethrin, Bioallethrin((S)-cyclopentylisomer), Bioresmethrin, Biphenate, (RS)-1-Cyano-1-(6-phenoxy-2-pyridyl)-methyl-(1RS)-trans-3-(4-tert.butylphenyl)-2,2-dimethylcyclopropanecarboxylate (NCI 85193), Cycloprothrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin.

4. Aus der Gruppe der Amidine

Amitraz, Chlordimeform

5. Aus der Gruppe der Zinnverbindungen

Cyhexatin, Fenbutatinoxide

6. Sonstige

Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-diphenylthiophen (UBI-T 930), Chlorfentezine, Cyclopropancarbonsäure-(2-naphthylmethyl)ester (Ro 12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)propyl)dimethylsilan, Fenoxycarb, 2-Fluoro-5-(4-(4-ethoxyphenyl-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramechylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 95 Gew.-% Wirkstoff, vorzugsweise zwischen 0,001 und 1 Gew.-% liegen. Die Anwendung geschieht in einer den Anwendungsformen angepaßten üblichen Weise.

Nachfolgende Beispiele dienen zur Erläuterung der Erfindung, ohne daß diese darauf beschränkt wäre.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 EO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

C. Chemische Beispiele

Beispiel 1

3-(4-Chlorphenyl)-3-(3,4-dimethoxyphenyl)-N-methoxy-N-methyl-acrylamid

Zu einer Lösung von 10,0 g 3-(4-Chlorphenyl)-3-(3,4-dimethoxyphenyl)-acrylsäure in 100 ml Tetrahydrofuran wurden 7,3 ml N-Methylmorpholin gegeben. Anschließend wurde auf -15 °C abgekühlt und es wurden 3,35 ml Chlorameisensäureethylester zugetropft. Es wurde 20 min. bei dieser Temperatur gerührt und anschließend auf -60 °C abgekühlt und 3,07 g N-O-Dimethylhydroxylamin-hydrochlorid zugesetzt. Es wurde 2 Stunden bei -60 °C gerührt und man ließ auf Raumtemperatur kommen. Es wurde auf wäßrige Ammoniumchlorid-Lösung gegeben, mehrfach mit Ether extrahiert, die organischen Phasen mit gesättigter NaCl-Lösung gewaschen und über Magnesiumsulfat getrocknet. Das Lösungsmittel wurde im Vakuum abgedampft und der Rückstand chromatographisch gereinigt (Eluent Ethylacetat). Es wurden 3,8 g eines Diastereomeren (1a) und 1,4 g des anderen Diastereomeren (1b) erhalten in Form farbloser Öle erhalten.

$^1$H-NMR (CDCl3): 6,67 ppm (Olefin-H), 1a
6,53 ppm (Olefin-H), 1b

Die Zuordnung der absoluten Stereochemie der Isomere gelingt mit Hilfe der NMR-Spektroskopie unter Zuhilfenahme des Kern-Overhauser-Effektes (The Nuclear Overhauser Effect, J.H.Noggle, R.E.Schirmer, Academic Press New York und London 1971). Dabei ergibt sich, daß das Isomer 1a der absoluten Konfiguration "E" und das Isomer 1b der absoluten Konfiguration "Z" zugeordnet werden kann.

In entsprechender Weise werden die Verbindungen der Tabelle 1 hergestellt.

Tabelle 1

| Beispiel Nr. | $R^1$ | $R^2$ | phys. Daten $n^D_{30}$/Fp |
|---|---|---|---|
| 1a (E) | $CH_3$ | $CH_3$ | 1,5830 |
| 1b (Z) | $CH_3$ | $CH_3$ | 1,5480 |
| 2 | $CH_3$ | $C_2H_5$ | 1,5825 |
| 3 | $CH_3$ | $n\text{-}C_3H_7$ | |
| 4 | $CH_3$ | $n\text{-}C_4H_9$ | |
| 5 | $CH_3$ | $CH_2\text{-}CH_2\text{-}OCH_3$ | |
| 6 | $CH_3$ | $CH_2\text{-}CH_2\text{-}O\text{-}C_2H_5$ | |
| 7 | $CH_3$ | $CH_2\text{-}CH=CH_2$ | Wachs |
| 8 | $CH_3$ | $CH_2\text{-}CH=CH\text{-}CH_3$ | |
| 9 | $CH_3$ | $CH_2\text{-}CH=CH\text{-}OCH_3$ | |
| 10 | $CH_3$ | $CH_2\text{-}C(OCH_3)=CH_2$ | |
| 11 | $CH_3$ | $CH_2\text{-}C\equiv CH$ | 1,5950 |
| 12 | $CH_3$ | $CH_2\text{-}C\equiv C\text{-}Si(CH_3)_3$ | |
| 13 | $CH_3$ | $CH_2\text{-}C\equiv C\text{-}CH_3$ | |
| 14 | $CH_3$ | $CH(CH_3)\text{-}C\equiv CH$ | |
| 15 | $CH_3$ | $CH_2\text{-}C_6H_5$ | 1,6050 |
| 16 | $CH_3$ | $CH_2\text{-}C_6H_4\text{-}4\text{-}CH_3$ | |
| 17 | $CH_3$ | $CH_2\text{-}C_6H_4\text{-}4\text{-}F$ | |
| 18 | $CH_3$ | $CH_2\text{-}C_6H_4\text{-}2\text{-}Cl$ | |
| 19 | $CH_3$ | $CH_2\text{-}C_6H_3\text{-}3,4\text{-}(Cl)_2$ | |

| Beispiel Nr. | $R^1$ | $R^2$ | phys. Daten $n^D_{30}$/Fp |
|---|---|---|---|
| 20 | $CH_3$ | $CH_2$-$C_6H_4$-4-$OCH_3$ | |
| 21 (Z) | $C_2H_5$ | $CH_3$ | 1,5751 |
| 22 | $C_2H_5$ | $C_2H_5$ | |
| 23 (Z) | $C_2H_5$ | $CH_2$-CH=$CH_2$ | 1,5830 |
| 24 | $C_2H_5$ | $CH_2$-CH=CH-$OCH_3$ | |
| 25 | $C_2H_5$ | $CH_2$-C≡C-H | 1,5920 |
| 26 | $C_2H_5$ | $CH_2$-C≡C-Si$(CH_3)_3$ | |
| 27 | $C_2H_6$ | $CH_2$-$C_6H_4$-4-$SO_2CH_3$ | |
| 28 | $C_2H_5$ | $CH_2$-C≡C-$CH_2$-$OCH_3$ | |
| 29 | $C_2H_5$ | CH($CH_3$)-$C_6H_5$ | |
| 30 | $CH_2CF_3$ | $CH_3$ | |
| 31 | $CH_2CF_3$ | $CH_2CF_3$ | |
| 32 | $CH_2CF_3$ | $CH_2$-CH=$CH_2$ | |
| 33 | $CH_2CF_3$ | $CH_2$-$C_6H_4$-3-O-$C_6H_5$ | |
| 34 | $CH_2CF_3$ | $CH_2$-$C_6H_4$-3-$CF_3$ | |
| 35 | $CH_2CF_3$ | $CH_2$-$CH_2$-$OCH_3$ | Öl |
| 36 | $CF_2$-$CHF_2$ | $CH_2$-$CH_2$-O-$C_2H_5$ | |
| 37 | $CF_2$-$CHF_2$ | $CH_3$ | |
| 38 | $CF_2$-$CHF_2$ | $CF_2$-$CHF_2$ | |
| 39 | $CF_2$-$CHF_2$ | $CH_2$-$C_6H_5$ | |
| 40 | $CF_2$-$CHF_2$ | $CH_2$-CH=$CH_2$ | |

| Beispiel Nr. | $R^1$ | $R^2$ | phys. Daten $n^D{}_{30}$/Fp |
|---|---|---|---|
| 41 | n-$C_3H_7$ | $CH_3$ | |
| 42 | n-$C_3H_7$ | $C_2H_5$ | |
| 43 | n-$C_3H_7$ | $CH_2CH(CH_3)_2$ | |
| 44 | n-$C_3H_7$ | $CH_2$-$CH=CH_2$ | |
| 45 | n-$C_3H_7$ | $CH_2$-$C\equiv C$-$Si(CH_3)_3$ | |
| 46 | n-$C_3H_7$ | n-$C_3H_7$ | |
| 47 | n-$C_3H_7$ | $CH(CH_3)$-$C_6H_5$ | |
| 48 | n-$C_4H_9$ | $CH_3$ | |
| 49 | n-$C_4H_9$ | n-$C_4H_9$ | |
| 50 | n-$C_4H_9$ | $CH_2$-$CH(CH_3)_2$ | |
| 51 | n-$C_4H_9$ | $CH_2$-$CH=CH_2$ | |
| 52 | n-$C_4H_9$ | $CH_2$-$C\equiv C$-$CH_3$ | |
| 53 | n-$C_4H_9$ | $CH_2$-$C_6H_4$-2-Br | |
| 54 | n-$C_4H_9$ | $CH_2$-$C_6H_3$-2,4-$F_2$ | |
| 55 | i-$C_4H_9$ | $CH_3$ | |
| 56 | i-$C_4H_9$ | $C_2H_5$ | |
| 57 | i-$C_4H_9$ | i-$C_4H_9$ | |
| 58 | i-$C_4H_9$ | $CH_2$-$C_6H_5$ | |
| 59 | $CH_2$-$OCH_3$ | $CH_3$ | |
| 60 | $CH_2$-$OCH_3$ | $C_2H_5$ | |
| 61 | $CH_2$-$OCH_3$ | $CH_2$-$CH=CH_2$ | |

| Beispiel Nr. | $R^1$ | $R^2$ | phys. Daten $n^D{}_{30}$/Fp |
|---|---|---|---|
| 62 | $CH_2$-$OCH_3$ | $CH_2$-C≡CH | |
| 63 | $CH_2$-$OCH_3$ | $CH_2$-$C_6H_5$ | |
| 64 | $CH_2$-O-$CH_3$ | $CH_2$-$OCH_3$ | |
| 65 | $CH_2$-$OCH_3$ | $CH_2F$ | |
| 66a (E) | $CH_2$-CH=$CH_2$ | $CH_3$ | 1,5889 |
| 66b (Z) | $CH_2$-CH=$CH_2$ | $CH_3$ | 1,5878 |
| 67a (E) | $CH_2$-CH=$CH_2$ | $C_2H_5$ | Harz |
| 67b (Z) | $CH_2$-CH=$CH_2$ | $C_2H_5$ | Harz |
| 68 | $CH_2$-CH=$CH_2$ | n-$C_3H_7$ | |
| 69 | $CH_2$-CH=$CH_2$ | $CH_2$-$CH(CH_3)_2$ | |
| 70a (E) | $CH_2$-CH=$CH_2$ | $CH_2$-CH=$CH_2$ | 1,5904 |
| 70b (Z) | $CH_2$-CH=$CH_2$ | $CH_2$-CH=$CH_2$ | 1,5714 |
| 71 | $CH_2$-CH=$CH_2$ | $CH_2$-C≡C-$Si(CH_3)_3$ | |
| 72(E/Z) | $CH_2$-CH=$CH_2$ | $CH_2$-$C_6H_5$ | 1,5932 |
| 73 | $CH_2$-CH=$CH_2$ | $CH_2$-$C_6H_4$-3-F | |
| 74 | $CH_2$-CH=$CH_2$ | $CH_2$-$C_6H_4$-3-$OC_6H_5$ | |
| 75 | $CH_2$-CH=$CH_2$ | $CH_2$-$C_6H_4$-4-$OCH_3$ | |
| 76 | $CH_2$-CH=$CH_2$ | $CH_2$-$C_6H_4$-3-$CF_3$ | |
| 77 | $CH_2$-CH=$CH_2$ | $CH_2$-$C_6H_4$-4-Cl | |
| 78 | $CH_2$-CH=$CH_2$ | $CH_2$-$C_6H_3$-2,4-$Cl_2$ | |
| 79 | $CH_2$-CH=$CH_2$ | $CH_2$-$C_6H$-2,3,5,6-$F_4$ | |

| Beispiel Nr. | $R^1$ | $R^2$ | phys. Daten $n^D_{30}$/Fp |
|---|---|---|---|
| 80 | $CH_2$-CH = $CH_2$ | $CH_2$-$C_6F_4$-4-$CH_3$ | |
| 81 | $CH_2$-C≡CH | $CH_3$ | |
| 82 | $CH_2$-C≡CH | $C_2H_5$ | |
| 83 | $CH_2$-C≡CH | $CH_2$-C≡CH | |
| 84 | $CH_3$-C≡CH | $CH_2$-$C_6H_5$ | |
| 85 | $CH_2$-C≡CH | $CH_2OCH_3$ | |
| 86 | $CH_2$-C≡CH | $CH_2OCH_2$-$CH_2$-$CH_3$ | |
| 87 | $CH_2$-C≡CH | $CH_2CH_2OC_2H_5$ | |
| 88 | $CH_2$-C≡C-Si$(CH_3)_3$ | $CH_3$ | |
| 89(E/Z) | $CH_2$-$C_6H_5$ | $CH_3$ | 102-103°C |
| 90 | $CH_2$-$C_6H_5$ | $CHF_2$ | |
| 91 | $CH_2$-$C_6H_5$ | $CH_2Cl$ | |
| 92(E/Z) | $CH_2$-$C_6H_5$ | $CH_2$-CH = $CH_2$ | Harz |
| 93 | $CH_2$-$C_6H_5$ | CH$(CH_3)$-CH = $CH_2$ | |
| 94 | $CH_2$-$C_6H_5$ | $CH_2$-C$(CH_3)$ = $CH_2$ | |
| 95 | $CH_2$-$C_6H_5$ | $CH_2$-C≡CH | |
| 96(E/Z) | $CH_2$-$C_6H_5$ | $C_2H_5$ | Harz |
| 97 | $CH_2$-$C_6H_5$ | n-$C_3H_7$ | |
| 98 | $CH_2$-$C_6H_5$ | i-$C_3H_7$ | |
| 99 | $CH_2$-$C_6H_5$ | $CH_2CF_3$ | |
| 100 | $CH_2$-$C_6H_5$ | $CF_2$-CHF-$CF_3$ | |

| Beispiel Nr. | $R^1$ | $R^2$ | phys. Daten $n^D{}_{30}$/Fp |
|---|---|---|---|
| 101 | $CH_2$-$C_6H_4$-4-F | $CH_3$ | |
| 102 | $CH_2$-$C_6H_4$-4-F | $CH_2$-$CH=CH_2$ | |
| 103 | $CH_2$-$C_6H_4$-4-F | $CH_2$-$C\equiv CH$ | |
| 104 | $CH_2$-$C_6H_4$-4-F | $CH_2$-$C_6H_4$-4-F | |
| 105 | $CH_2$-$C_6H_4$-4-$OCH_3$ | $CH_3$ | |
| 106 | $CH_2$-$C_6H_4$-4-$OCH_3$ | $C_2H_5$ | |
| 107 | $CH_2$-$C_6H_4$-4-$OCH_3$ | $CH_2$-$C_6H_5$ | |
| 108 | $CH_2$-$C_6H_4$-3-$CF_3$ | $CH_3$ | |
| 109 | $CH_2$-$C_6H_4$-3-$CF_3$ | $CH_2$-$CH_2$-$OC_2H_5$ | |
| 110 | $CH_2$-$C_6H_4$-4-$CH_3$ | $CH_3$ | |
| 111 | $CH_2$-$C_6H_4$-4-$CH_3$ | $CH_2$-$C_6H_4$-4-$CH_3$ | |
| 112 | $CH_2$-$C_6H_4$-4-$SO_2CH_3$ | $CH_3$ | |
| 113 | $CH_2$-$C_6H_4$-4-$SO_2CH_3$ | $C_2H_5$ | |
| 114 | $CH_2$-$C_6H_4$-4-$COOC_2H_5$ | $CH_3$ | |
| 115 | $CH_2$-$C_6H_4$-4-$COOC_2H_5$ | $CH_2$-$C_6H_4$-4-$COOC_2H_5$ | |
| 116 | $CH_2$-$C_6H_3$-4-Cl-3-F | $CH_3$ | |
| 117 | $CH_2$-$C_6H_3$-3-Cl-4-F | $CH_2$-$C_6H_3$-3-Cl-4-F | |
| 118 | $CH_2$-$C_6F_4H$ | $CH_2C_6F_4H$ | |
| 119 | $CH_2$-$C_6F_4$-4-$CH_3$ | $CH_2$-$C_6F_4$-4-$CH_3$ | |
| 120 | $CH_2$-$C_6H_4$-2-CN | $CH_3$ | |

C. Biologische Beispiele

Als Vergleichsstoff wurde N-Methoxy-3-(4-methoxy-3-methylphenyl)-N-methyl-zimtsäureamid der Formel VIII verwendet:

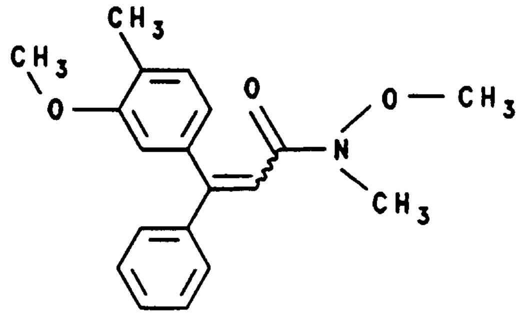

VIII

Beispiel 1: Phytophthora infestans

Tomatenpflanzen der Sorte "Rheinlands Ruhm" wurden im 3 bis 4-Blattstadium mit wäßrigen Suspensionen der beanspruchten Verbindungen gleichmäßig tropfnaß benetzt. Nach dem Antrocknen wurden die Pflanzen mit einer Zoosporangien-Suspension von Phytophthora infestans inokuliert und für 2 Tage unter optimalen Infektionsbedingungen in einer Klimakammer gehalten. Danach wurden die Pflanzen bis zur Symptomausprägung im Gewächshaus weiterkultiviert. Die Befallsbonitur erfolgte ca. 1 Woche nach Inokulation. Der Befallsgrad der Pflanzen wurden in % befallener Blattfläche im Vergleich zu den unbehandelten, zu 100 % infizierten Kontrollpflanzen ausgedrückt. Bei 500 mg Wirkstoff/l Spritzbrühe zeigen die folgenden Substanzen eine vollständige Befallsunterdrückung:

1a, 1b

Im Vergleich hierzu zeigt VIII keine Wirkung.

Beispiel 2: Plasmopara viticola

Weinsämlinge der Sorten "Riesling/Ehrenfelder" wurden ca. 6 Wochen nach der Aussaat mit wäßrigen Suspensionen der beanspruchten Verbindungen tropfnaß behandelt. Nach dem Antrocknen des Spritzbelages wurden die Pflanzen mit einer Zoosporangiensuspension von Plasmopara viticola inokuliert und tropfnaß für 4 - 5 h in eine Klimakammer mit 23 °C und 80 - 90 % rel. Luftfeuchte gestellt.

Nach einer Inkubationszeit von 7 Tagen im Gewächshaus wurden die Pflanzen nochmals über Nacht in die Klimakammer gestellt, um die Sporulation des Pilzes anzuregen. Anschließend erfolgte die Befallsauswertung. Der Befallsgrad wurde in % befallener Blattfläche im Vergleich zu den unbehandelten, zu 100 % infizierten Kontrollpflanzen ausgedrückt.

Bei 500 mg Wirkstoff/l Spritzbrühe zeigen die folgenden Substanzen eine vollständige Befallsunterdrükkung:

1a, 1b

Im Vergleich hierzu zeigt VIII keine Wirkung.

**Patentansprüche**

**1.** Verbindung der Formel I,

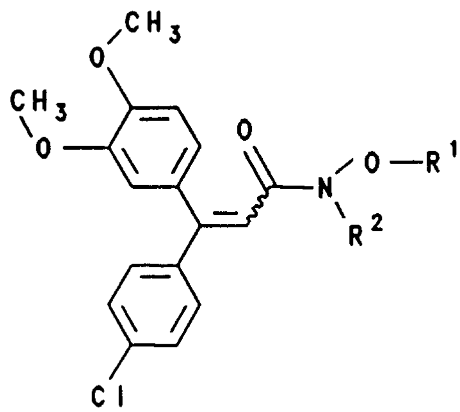

(I)

in welcher
$R^1$ und $R^2$ unabhängig voneinander $C_1$-$C_6$-Alkyl, $C_2$-$C_6$-Alkenyl, $C_2$-$C_6$-Alkinyl, $C_1$-$C_4$-Alkoxy-$C_1$-$C_6$-alkyl, $C_1$-$C_4$-Alkoxy-$C_2$-$C_6$-alkenyl, $C_1$-$C_4$-Alkoxy-$C_2$-$C_6$-alkinyl, Tri-($C_1$-$C_4$-alkyl)-silyl-$C_2$-$C_6$-alkinyl oder Aryl-$C_1$-$C_2$-alkyl, wobei Aryl gegebenenfalls bis zu vierfach durch gleiche oder verschiedene Reste aus der Reihe $C_1$-$C_4$-Alkyl, $C_1$-$C_4$-Alkoxy, $C_1$-$C_4$-Haloalkyl, $C_1$-$C_4$-Haloalkoxy, $C_1$-$C_4$-Alkoxycarbonyl, Halogen, Cyano, Nitro, $C_1$-$C_4$-Alkylsulfonyl, Phenyl und Phenoxy substituiert ist,
bedeuten,

**2.** Verbindung der Formel I gemäß Anspruch 1, in welcher $R^1$ und $R^2$ unabhängig voneinander $C_1$-$C_4$-Alkyl, $C_2$-$C_4$-Alkenyl, $C_2$-$C_4$-Alkinyl, $C_1$-$C_4$-Alkoxy-$C_1$-$C_4$-alkyl, $C_1$-$C_4$-Alkoxy-$C_2$-$C_4$-alkenyl, $C_1$-$C_4$-Alkoxy-$C_2$-$C_4$-alkinyl, Tri-($C_1$-$C_2$-Alkyl)-silyl-$C_2$-$C_4$-alkinyl oder Aryl-methyl, wobei Aryl gegebenenfalls bis zu vierfach substituiert ist durch gleiche oder verschiedene Reste aus der Reihe $C_1$-$C_4$-Alkyl, $C_1$-$C_2$-Alkoxy, $C_1$-$C_4$-Haloalkyl, $C_1$-$C_2$-Haloalkoxy, $C_1$-$C_2$-Alkoxycarbonyl, Halogen, Cyano, Nitro, Methylsulfonyl, Phenyl und Phenoxy, bedeuten.

**3.** Verbindung der Formel I gemäß Anspruch 1 oder 2, in welcher $R^1$ und $R^2$ unabhängig voneinander $C_1$-$C_2$-Alkyl, $C_2$-$C_3$-Alkenyl, $C_2$-$C_3$-Alkinyl, $C_1$-$C_2$-Alkoxy-$C_1$-$C_2$-alkyl, $C_1$-$C_2$-Alkoxy-$C_2$-$C_3$-alkenyl, $C_1$-$C_2$-Alkoxy-$C_2$-$C_3$-alkinyl, Trimethylsilyl-$C_2$-$C_3$-alkinyl oder Benzyl bedeuten.

**4.** Verfahren zur Herstellung einer Verbindung der Formel I gemäß einem der Ansprüche 1 bis 3, dadurch gekennzeichnet, daß man
a) 3-(4-Chlorphenyl)-3-(3,4-dimethoxyphenyl)-acrylsäure der Formel II oder

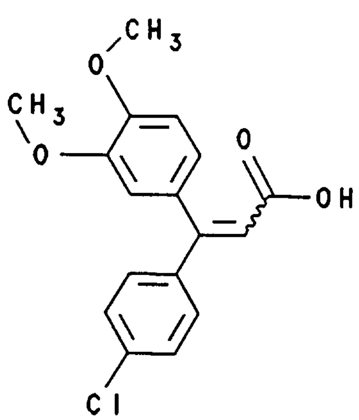

(II)

ein gegebenenfalls daraus in situ hergestelltes reaktionsfähiges Derivat umsetzt mit einem Hydroxyaminderivat der Formel III,

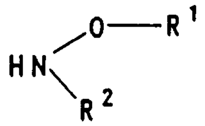

(III)

in dem $R^1$ und $R^2$ wie zuvor definiert sind und zusätzlich $R^1$ und/oder $R^2$ Wasserstoff bedeuten können;

b) ein Keton der Formel IV umsetzt mit einem Phosphonessigsäureamid der Formel V,

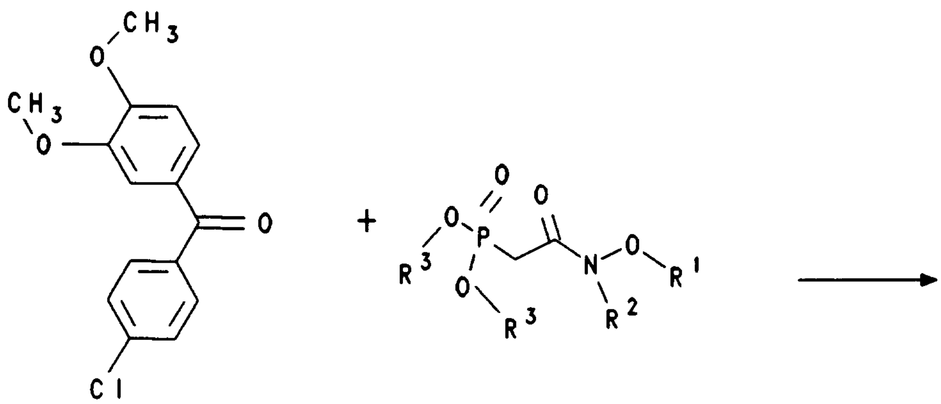

(I)

(IV) (V)

in dem $R^1$ und $R^2$ wie oben definiert sind und $R^3$ für einen aliphatischen Rest steht;

c) ein N-Alkoxyzimtsäureamid der Formel VI in Gegenwart einer Base mit einem Alkylierungsmittel der Formel VII, wobei die Reste $R^1$ und $R^2$ wie oben definiert sind und X Halogen oder Alkoxysulfonyl bedeutet;

oder

d) ein Keton der Formel IV in Gegenwart einer starken Base umsetzt mit einem Acethydroxamsäurederivat der allgemeinen Formel IX, wobei die Reste $R^1$ und $R^2$ wie oben definiert sind.

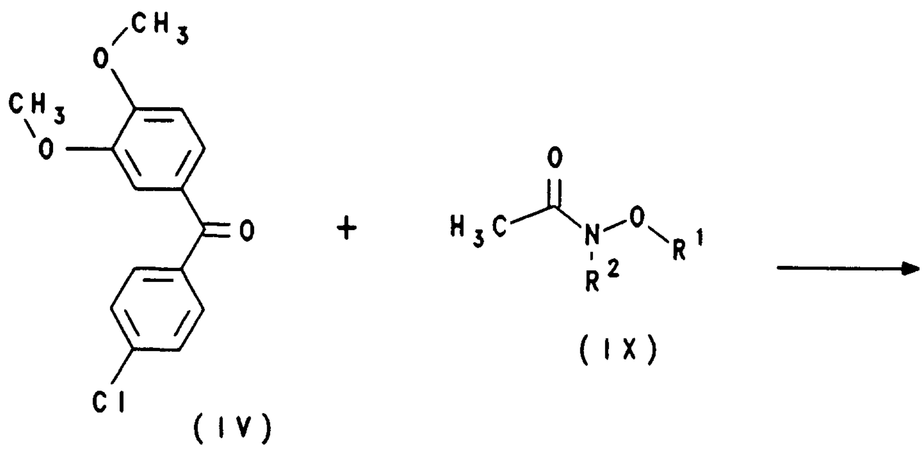

I

(IX)

(IV)

**5.** Mittel, enthaltend mindestens eine Verbindung gemäß einem der Ansprüche 1 bis 3 und mindestens ein Formulierungsmittel.

**6.** Fungizide Mittel gemäß Anspruch 5, enthaltend eine fungizid wirksame Menge mindestens einer Verbindung gemäß einem der Ansprüche 1 bis 3 zusammen mit den für deren Anwendung üblichen Zusatz- oder Hilfsstoffen.

7. Pflanzenschutzmittel, enthaltend eine fungizid wirksame Menge mindestens einer Verbindung gemäß einem der Ansprüche 1 bis 3 und mindestens einem weiteren Wirkstoff, vorzugsweise aus der Reihe der Fungizide, Insektizide, Lockstoffe, Sterilanten, Akariziden, Nematiziden und Herbizide zusammen mit den für diese Anwendung üblichen Hilfs- und Zusatzstoffen.

8. Mittel zur Anwendung im Holzschutz oder als Konservierungsmittel in Anstrichfarben, in Kühlschmiermitteln für die Metallbearbeitung oder in Bohr- und Schneidölen, enthaltend eine wirksame Menge mindestens einer Verbindung gemäß einem der Ansprüche 1 bis 3 zusammen mit den für diese Anwendungen üblichen Hilfs- und Zusatzstoffen.

9. Verfahren zur Herstellung eines Mittels gemäß einem der Ansprüche 5 bis 8, dadurch gekennzeichnet, daß man den Wirkstoff und die weiteren Zusätze zusammen gibt und in eine geeignete Anwendungsform bringt.

10. Verwendung einer Verbindung gemäß einem der Ansprüche 1 bis 3 oder eines Mittels gemäß einem der Ansprüche 5 bis 8 als Fungizid.

11. Verwendung einer Verbindung gemäß einem der Ansprüche 1 bis 3 oder eines Mittels gemäß einem der Ansprüche 5, 6 und 8 als Holzschutzmittel oder als Konservierungsmittel in Anstrichfarben, in Kühlschmiermitteln für die Metallbearbeitung oder in Bohr- und Schneidölen.

12. Verfahren zur Bekämpfung von phytopathogenen Pilzen, dadurch gekennzeichnet, daß man auf diese oder die von ihnen befallenen Pflanzen, Flächen oder Substrate oder auf Saatgut eine fungizid wirksame Menge einer Verbindung gemäß einem der Ansprüche 1 bis 3 oder eines Mittels gemäß einem der Ansprüche 5 bis 7 appliziert.

Europäisches Patentamt

# EUROPÄISCHER RECHERCHENBERICHT

Nummer der Anmeldung: EP 93119421.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.5) |
|---|---|---|---|
| D,X | EP - A - 0 208 999 (CELAMERCK) * Ansprüche; Verbindungen 69-71 * -- | 1-6,9, 10,12 | C 07 C 259/06<br>A 01 N 37/28 |
| A | EP - A - 0 046 931 (BAYER AG) * Ansprüche 1,5-8 * ---- | 1,5,6, 9,10, 12 | |

RECHERCHIERTE SACHGEBIETE (Int Cl.5)

C 07 C 259/00
A 01 N 37/00

Der vorliegende Recherchenbericht wurde für alle Patentansprüche erstellt.

| Recherchenort | Abschlußdatum der Recherche | Prüfer |
|---|---|---|
| WIEN | 18-02-1994 | KÖRBER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument